(19) **Europäisches Patentamt European Patent Office Office européen des brevets**

(11) **EP 4 446 003 A1**

(12) **EUROPEAN PATENT APPLICATION**
published in accordance with Art. 153(4) EPC

(43) Date of publication:
**16.10.2024 Bulletin 2024/42**

(21) Application number: **22909942.9**

(22) Date of filing: **19.12.2022**

(51) International Patent Classification (IPC):
**B01J 19/08** (2006.01)    **C07C 2/80** (2006.01)
**C07C 11/04** (2006.01)    **H01J 37/32** (2006.01)
**C23C 16/455** (2006.01)    **C23C 16/40** (2006.01)
**C07C 11/24** (2006.01)    **C07C 2/84** (2006.01)
**B01J 19/00** (2006.01)    **H05H 1/34** (2006.01)
**B01J 23/89** (2006.01)    **B01J 23/42** (2006.01)
**B01J 23/44** (2006.01)    **B01J 23/46** (2006.01)
**B01J 23/50** (2006.01)    **B01J 23/52** (2006.01)
**B01J 29/44** (2006.01)    **B01J 23/745** (2006.01)

(52) Cooperative Patent Classification (CPC):
**B01J 19/00; B01J 19/08; B01J 23/42; B01J 23/44;**
**B01J 23/46; B01J 23/50; B01J 23/52;**
**B01J 23/745; B01J 23/89; B01J 29/44; C07C 2/80;**
**C07C 2/84; C07C 11/04; C07C 11/24; C23C 16/40;**

(Cont.)

(86) International application number:
**PCT/CN2022/140057**

(87) International publication number:
**WO 2023/116630 (29.06.2023 Gazette 2023/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:   20.12.2021   CN 202111567211
20.12.2021   CN 202111565360
20.12.2021   CN 202111567220
20.12.2021   CN 202111567216
20.12.2021   CN 202111567218
20.12.2021   CN 202111565366
20.12.2021   CN 202111565369
20.12.2021   CN 202111567199
20.12.2021   CN 202111567219

(71) Applicants:
• **China Petroleum & Chemical Corporation**
  **Beijing 100728 (CN)**
• **Sinopec Research Institute of Safety**
  **Engineering Co., Ltd.**
  **Qingdao, Shandong 266104 (CN)**

(72) Inventors:
• **ZHANG, Jing**
  **Qingdao, Shandong 266104 (CN)**
• **XU, Wei**
  **Qingdao, Shandong 266104 (CN)**
• **YANG, Zhe**
  **Qingdao, Shandong 266104 (CN)**
• **ZHOU, Mingchuan**
  **Qingdao, Shandong 266104 (CN)**
• **REN, Junpeng**
  **Qingdao, Shandong 266104 (CN)**
• **ZHANG, Tie**
  **Qingdao, Shandong 266104 (CN)**
• **SUN, Feng**
  **Qingdao, Shandong 266104 (CN)**
• **JIANG, Jie**
  **Qingdao, Shandong 266104 (CN)**
• **SUN, Bing**
  **Qingdao, Shandong 266104 (CN)**

(74) Representative: **karo IP**
**Patentanwälte PartG mbB**
**Steinstraße 16-18**
**40212 Düsseldorf (DE)**

(54) **GLIDING ARC PLASMA REACTOR, AND METHOD FOR CONVERTING METHANE BY MEANS OF PLASMA**

EP 4 446 003 A1

(57) The present invention relates to the field of energy chemical industry, and disclosed are a gliding arc plasma reactor, and a method for converting methane by means of plasma. The reactor comprises a reactor chamber and a gliding arc plasma generator arranged in the reactor chamber, wherein the gliding arc plasma generator comprises at least two arc surface electrodes which are symmetrically distributed, such that a discharge area can be formed between the arc surface electrodes. According to the gliding arc plasma reactor provided in the present invention, the conversion rate of methane can be significantly improved when methane is converted into olefin; the selectivity of ethylene in the product is improved; and carbon deposition is significantly reduced. In addition, compared with a traditional process for preparing olefin from methane, no $CO_2$ is generated; the ignition and explosion risk is avoided; and the reactor is safer and more environmentally friendly.

Figure 1

(52) Cooperative Patent Classification (CPC): (Cont.)
**C23C 16/455; H01J 37/32; H05H 1/34**

**Description**

**Cross Reference to Related Applications**

The present application claims the benefits of Chinese patent applications 202111567211.X, 202111565360.2, 202111567220.9, 202111567216.2, 202111567218.1, 202111565366.X, 202111565369.3, 202111567199.2, 202111567219.6 filed on 20/12/2021, 20/12/2021, 20/12/2021, 20/12/2021, 20/12/2021, 20/12/2021, 20/12/2021, 20/12/2021, 20/12/2021, the contents of which are incorporated herein by reference.

**Field of the invention**

The present invention relates to the field of energy chemical industry, in particular to a gliding arc plasma reactor, and a method for converting methane by means of plasma.

**Background of the invention**

The plasma methane conversion technology is researched from the last 80 years in China, and gradually forms a patent technology from 2000.

CN100999432A discloses a method for preparing C2 hydrocarbons by ionic liquid catalytic plasma methane conversion, and the patent rights were terminated in 2015.

CN101734620A discloses a method for producing hydrogen by methane-rich gas plasma, and the patent rights were terminated in 2014.

The southwest chemical research and design institute has published a series of plasma methane cracking patent technologies (CN 210367505U, CN109294284A, CN106478332A and CN 101921163A), and the technology is mainly developed aiming at the technology of preparing carbon black or acetylene and hydrogen by converting methane through plasma, and particularly emphasizes on the design and optimization of the technology.

The technology for preparing acetylene by plasma cracking coal (CN 203582763U, CN102068953A, CN101734620A, CN101550057A, CN101734995A and CN 1613839A) is jointly developed by Tsinghua University, Taiyuan University of Technology and Xinjiang Tianye Co.,Ltd., coal is mainly used as a raw material, natural gas is used for preparing acetylene and hydrogen in an auxiliary way, and the working gas is hydrogen.

Zhejiang University is mainly developed aiming at a plasma online decoking method (CN 104056828A and CN 104056829A), and $CO_2$ or $H_2$ can be introduced to remove carbon on the surface of an electrode. A rotating arc plasma is also developed to crack methane to prepare acetylene (CN 103333044A, CN 101844744A), working gas rotates to enter a discharge gap, and simultaneously, millisecond-level cracking occurs by adopting magnetic field driving from the outside.

At present, foreign related researches show that hydrocarbon products formed by converting methane by means of plasma mainly belong to two types, wherein one type mainly comprises alkane such as ethane and the like, and the other type mainly comprises acetylene.

Researchers have found that the product distribution can be adjusted by varying the feed gas flow or by incorporating inert gases. The technology has been industrialized abroad, and comprises four processes: the HUELS method, the AVCO method, the Du Pont method, and the Romania method.

Through literature comparison, the utilization of electric arc to generate high-temperature cracked natural gas to generate acetylene has low electric energy utilization rate, 13900kWh is consumed for producing 1 ton of acetylene, and the cost is more than 50% of the total cost, so that the purpose of saving energy and reducing consumption is achieved by changing the structure of a reactor, and the method is one of the key points for innovation in foreign patent literature.

On the basis of the technology, a series of 'warm' plasma technology and 'cold' plasma technology are gradually developed in the follow-up process, energy consumption is reduced by changing an energy generation form, and a catalyst is added for coupling, so that methane is directionally converted into a target product. This process is currently still under investigation.

Thanyachotpaiboon et al (Conversion of methane to high hydrocarbons in AC nonequilibrium plasmas [J]. AIChE Journal, 1998, 44(10): 2252-7.) examined the conversion of methane using DBD discharge at room temperature and examined the effect of adding He and $C_2H_6$ on the conversion of methane discharge. When only $CH_4$ is used as a reactant, as the discharge voltage increases (6→11kV), the $CH_4$ conversion rate increases and the product selectivity does not change much: the products are mainly $C_2H_6$ and $C_3H_8$; the conversion of $CH_4$, obtained at a flow rate of $CH_4$ of 20mL/min and a discharge voltage of 11kV, is about 23%, and the selectivities of the products $C_2H_6$, $C_3H_8$, $C_4H_{10}$ and $C_2H_4$ were 40%, 15%, 5% and 2%, respectively.

RUEANGJITT N et al (Non-oxidative reforming of methane in a mini-gliding arc discharge reactor: Effects of feed methane concentration, feed flow rate, electrode gap distance, residence time, and catalyst distance [J]. Plasma Chem Plasma Process, 2011, 31( 4) : 517-534.) use a gliding blade arc to convert methane, with acetylene as the main product, 40% -50% methane conversion and 20% $C_2H_2$ selectivity at a power of 110-190W.

However, there are currently no existing technical reports on the direct conversion of methane to olefins by means of plasma.

**Description of the invention**

The present invention aims to overcome the defect of low conversion efficiency of directionally converting methane into olefin in the prior art.

In order to achieve the above object, the first aspect of the present application provides a gliding arc plasma reactor comprising a reactor chamber and a gliding arc plasma generator disposed in the reactor chamber;

the gliding arc plasma generator comprises at least two arc surface electrodes (3) which are symmetrically distributed, the discharge surfaces of each arc surface electrode are all arc surface structures, the central angle corresponding to each arc surface electrode is $\alpha$, wherein $360° \geq \alpha > 5°$ ; the arrangement positions of the arc surface electrodes enable a discharge area to be formed between the arc surface electrodes (3).

In the second aspect the present invention provides a method for converting methane by means of plasma, the method being carried out in a gliding arc plasma reactor as described in the first aspect, the method comprising:

introducing a reaction gas containing methane into the gliding arc plasma reactor under plasma discharge conditions to carry out a methane conversion reaction.

Compared with the prior art, the scheme provided by the present invention at least has the following advantages:

(1) the gliding arc plasma reactor provided by the present invention adopts the electrodes to form the discharge area, and the discharge arc forming point is arranged on the arc surface, so that more arc channels are formed, and the gliding arc plasma reactor has stronger reactant conversion capability;
(2) the gliding arc plasma reactor provided by the present invention can enable the raw material gas to pass through the discharge area formed by the electrodes more intensively, so that the gas flow passing through the discharge area is effectively increased, the conversion efficiency of reactants is improved, and the energy consumption is reduced;
(3) the gliding arc plasma reactor provided by the present invention can realize one-step conversion of methane to efficiently generate olefin at higher reactant conversion efficiency, can maintain the continuous and stable reaction, and compared with a traditional process for preparing olefin from methane, no $CO_2$ is generated; the ignition and explosion risk is avoided; and the reactor is safer and more environmentally friendly.

Additional features and advantages of the present invention will be set forth in the detailed description which follows.

**Brief Introduction of the Drawings**

Figure 1 is a schematic structural diagram of a preferred embodiment of a gliding arc plasma reactor according to the present invention.
Figure 2 is a schematic structural diagram of another preferred embodiment of the gliding arc plasma reactor provided by the present invention.

Description of the reference numerals

| 1 | Reactor inlet | 2 | Gas nozzle |
| 3 | Electrode | 4 | Lower reaction zone |
| 5 | Product outlet | 6 | Base |
| 7 | Active connection mechanism | | |

**Detailed Description of the Preferred Embodiments**

The endpoints of the ranges and any values disclosed herein are not limited to the precise range or value, and these ranges or values should be understood to encompass values close to these ranges or values. For numerical ranges, each range between its endpoints and individual point values, and each individual point value can be combined with each other to give one or more new numerical ranges, and such numerical ranges should be construed as specifically disclosed herein.

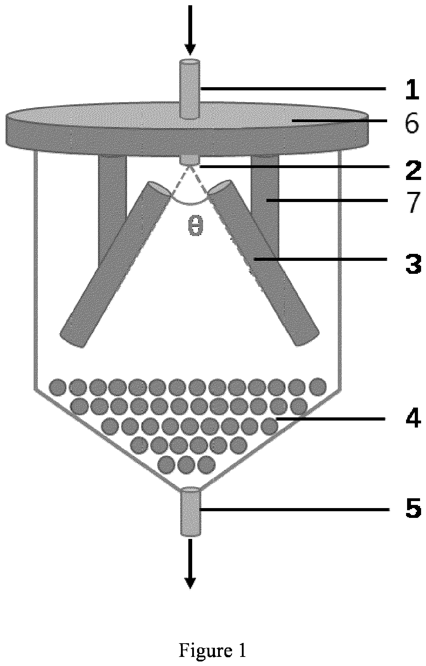

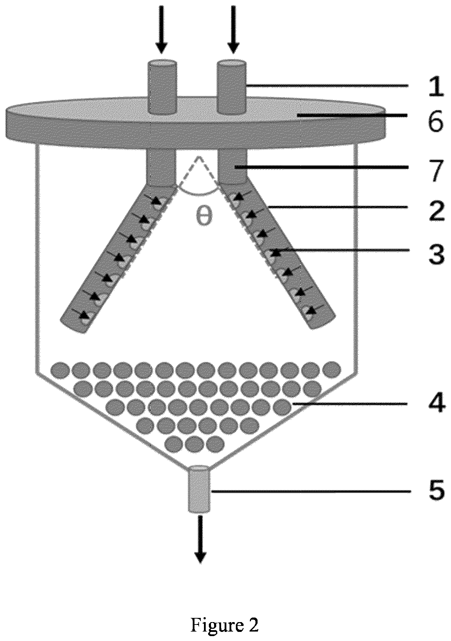

The embodiment of the present invention will be described in detail with reference to Figure 1 and Figure2.

As previously described, the first aspect of the present application provides a gliding arc plasma reactor comprising a reactor chamber and a gliding arc plasma generator disposed in the reactor chamber;

the gliding arc plasma generator comprises at least two arc surface electrodes 3 which are symmetrically distributed, the discharge surfaces of each arc surface electrode are all arc surface structures, the central angle corresponding to each arc surface electrode is $\alpha$, wherein $360\degree \geq \alpha > 5\degree$, preferred $360\degree \geq \alpha > 10\degree$; the arrangement positions of the arc surface electrodes enable a discharge area to be formed between the arc surface electrodes (3).

According to a particularly preferred embodiment, the reactor comprises a reactor inlet 1, a gliding arc plasma generator and a product outlet 5;

> the gliding arc plasma generator comprises at least two arc surface electrodes 3 which are symmetrically distributed, and the arrangement positions of the arc surface electrodes enable discharge areas to be formed among the arc surface electrodes (3);
> the gliding arc plasma generator further comprises a gas nozzle 2 and a base 6;
> the arc surface electrode 3 is arranged on the base 6; the gas nozzle 2 is arranged on the base 6 and/or the arc surface electrode 3, so that reaction gas can enter the gliding arc plasma reactor from the reactor inlet 1 through the gas nozzle 2;
> the central angle corresponding to the arc surface structure of arc surface electrode is $\alpha$, wherein $360\degree \geq \alpha > 5\degree$.

According to the present invention, the discharge arc forming point of the electrode is on the curved surface, so that more discharge areas of an arc channel can be formed, and the conversion capability of reactants is stronger.

Preferably, the arc surface structure of the arc surface electrode 3 is a semi-closed arc surface, the central angle corresponding to the arc surface is $\alpha$, wherein $270\degree > \alpha > 30\degree$; more preferably, the central angle $\alpha$ corresponding to the semi-closed arc surface is at least one selected from $180\degree$, $120\degree$, $90\degree$, $72\degree$, $60\degree$.

According to a preferred embodiment, the arc surface structure of the arc surface electrode 3 is a fully-closed arc surface. The inventor finds that when a fully-closed arc surface structure is used as the arc surface structure of the arc surface electrode 3, the conversion efficiency of the reactant is higher, and the heat dissipation is facilitated.

Preferably, the proportional relationship between the length d2 of the discharge area and the height d3 of the reactor chamber satisfies: 1: 1.2-1.8.

According to a preferred embodiment, the electrode gliding arc generator is the gliding arc plasma generator; and the center of the base of the gliding arc plasma generator is provided with the gas nozzle which is communicated with a gas inlet pipeline of the reactor inlet.

Preferably, the central angle corresponding to the arc surface structure of the arc surface electrode 3 is $\alpha$, wherein $360\degree \geq \alpha > 10\degree$.

Preferably, the gliding arc plasma generator comprises two arc surface electrodes 3 or six arc surface electrodes 3 which are symmetrically distributed.

Preferably, on each arc surface electrode 3, the proportional relation between the distance between two adjacent gas nozzles 2 and the inner diameter r2 of gas nozzle satisfies the following conditions: 1: 0.5-1.5.

Preferably, a proportional relationship between the inner diameter r2 of the gas nozzle 2 and the minimum width r3 of the discharge area satisfies: 1: 2-8.

According to a particularly preferred embodiment, the reactor of the present invention comprises a reactor inlet, an electrode gliding arc generator, a lower reaction zone and a product outlet;

> the electrode gliding arc generator comprises a gas nozzle, an electrode and a base;
> at least a plurality of electrodes which are symmetrically distributed are arranged on the base of the electrode gliding arc generator, so that a discharge area can be formed between the electrodes; the gas nozzle is arranged on the electrode and/or the base, so that reaction gas can enter the gliding arc plasma reactor from the reactor inlet through the gas nozzle;
> the structure of each two electrodes distributed symmetrically corresponds to each other to enable discharge to occur; and
> the electrode gliding arc generator is an electrode gliding arc generator with an arc surface electrode and/or a tubular electrode gliding arc generator with a tubular electrode.

Preferably, in the present invention, the symmetrical distribution is symmetrical distribution based on a central vertical axis of the base, and the mounting position of the electrode does not affect discharge.

In the present invention, the shape and material of the base are not particularly limited, and may be a circular shape or various other shapes that can achieve the aforementioned object of the present invention, and may be an insulating material or various other materials that can achieve the aforementioned object of the present invention.

Preferably, the gas nozzle 2 is arranged at the center of the base 6 of the gliding arc plasma generator, and the gas nozzle 2 is communicated with a gas inlet pipeline of the reactor inlet 1.

Preferably, the gas nozzle 2 is arranged in the relative direction of each two arc surface electrodes 3 in the symmetrical position, and the gas nozzle 2 is communicated with a gas inlet pipeline of the reactor inlet 1.

More preferably, at least a portion of the outer surface of the fully-closed arc surface structure is coated with a coating.

Preferably, the coating comprises at least two laminated plating layers of single-layer atomic layer deposition metal oxide thin films, the plating layers are made of semiconductor materials, the number of layers of the plating layer is at least two, and the coating materials for forming any two adjacent plating layers are different.

Preferably, the number of layers of the plating layer is at least three.

According to a preferable embodiment, the number of layers of the plating layer is three, and the coating materials forming the three plating layers are different; the dielectric constant of the coating material forming the outermost plating layer is 10-22$C^2$/(N.$M^2$) higher than that of the coating materials of the other plating layers.

Preferably, the coating material is an organic substance containing a metal element.

Preferably, the semiconductor material is a metal oxide; more preferably, the semiconductor material is selected from $Al_2O_3$, $ZrO_2$, $SnO_2$, $ZnO$, $HfO_2$, $TiO_2$, $La_2O_3$, $Ta_2O_5$, $Y_2O_3$.

Preferably, the outer surface of the fully-closed arc surface structure is coated by an atomic layer deposition method, and the coating step preferably comprises the following steps: in an atomic layer deposition apparatus,

(1) opening an ALD valve of a metal source tank in the presence of a carrier gas for coating, wherein a coating material in the metal source tank enters a reaction chamber containing oxygen, so that the coating material and the oxygen react on the surface of a blade electrode in the reaction chamber and form a plating unit, and the plating unit is a single-layer atomic layer deposition metal oxide thin film;

(2) repeating the step (1) to obtain a plating layer, repeatedly forming a single-layer atomic layer deposition metal oxide thin film each time, and adjusting the thickness of the plating layer by controlling the repetition times;

(3) repeating the above steps (1) to (2) using another coating material to form another plating layer.

Preferably, in the step (1), the temperature of the metal source tank is 140-160°C, the temperature of the reaction chamber is 50-400°C, and the temperatures of the transportation pipeline and the ALD valve are 180-200 °C.

Preferably, in step (1), the reaction chamber and the transport line are evacuated to a pressure of 10-200 Pa.

Preferably, in the step (1), the flow rate of the carrier gas for coating is 10-200 sccm.

Preferably, in step (1), the opening time of the ALD valve is 50-2000 ms.

Preferably, in the step (1), an inert gas pulse is introduced to clean the reaction chamber; preferably, the washing time is 1-200 s.

According to a preferred embodiment, the outer surface of the fully-closed arc surface structure is coated by an atomic layer deposition method, and the coating step preferably comprises the following steps: in an atomic layer deposition apparatus,

(1) placing a coating material in a metal source tank, and placing the blade electrode to be coated in a reaction chamber;

(2) heating the metal source tank, the reaction chamber, the transportation pipeline and the ALD valve, wherein the temperature of the metal source tank is 140-160°C, the temperature of the reaction chamber is 50-400°C, and the temperatures of the transportation pipeline and the ALD valve are 180-200°C, and vacuumizing the reaction chamber and the transportation pipeline to the pressure of 10-200 Pa;

(3) opening the carrier gas for coating of the metal source tank, wherein the flow rate of the carrier gas for coating is 10-200 sccm;

(4) opening the ALD valve of the metal source tank, wherein the opening time of the ALD valve is 50-2000ms, so that the coating material enters the reaction chamber, and oxygen is introduced into the reaction chamber, so that the coating material and the oxygen react on the surface of the blade electrode in the reaction chamber to form a plating unit, and the plating unit is a single-layer atomic layer deposition metal oxide thin film;

(5) cleaning the reaction chamber by introducing inert gas pulses, wherein the cleaning time is 1-200 s;

(6) repeating the steps (1) to (5) to obtain a plating layer, repeatedly forming a single-layer atomic layer deposition metal oxide film each time, and adjusting the thickness of the plating layer by controlling the repetition times;

(7) repeating the above steps (1) to (6) with another coating material to form another plating layer.

Preferably, the fully-closed arc surface is at least one selected from a tubular shape and a rod shape.

According to another preferred embodiment, the electrode gliding arc generator is the tubular electrode gliding arc generator; and the gas nozzles are arranged in the relative directions of every two tubular electrodes at the symmetrical positions and the gas nozzles are communicated with an inlet pipe of the reactor inlet.

Preferably, the material forming the gas nozzle is selected from at least one of a conductive material and an insulating material.

According to a preferred embodiment, the material forming the gas nozzle is an insulating material. The inventors have found that in this preferred case, the scheme of the present application can be performed more stably.

According to another preferred embodiment, the material forming the gas nozzle 2 is a conductive material, and the outlet position of the gas nozzle 2 does not overlap with the arc surface electrode 3 in the vertical direction.

Preferably, the electrical conductivity of the conductive material forming the gas nozzle 2 is >1MS/m, preferably >10MS/m; the thermal conductivity is >10W/(m.°C), preferably >50W/(m.°C).

Preferably, the material forming the electrodes is a conductive material.

More preferably, the conductive material forming the electrode is selected from at least one of 316L stainless steel, tungsten-cerium alloy, nickel-chromium alloy, zinc-copper alloy, copper-chromium alloy, nickel-copper alloy, cobalt-nickel alloy, cobalt-cadmium alloy, and graphite. The material forming the electrode can also be other high-temperature resistant and arc corrosion resistant conductive materials. The material forming the electrode can also be other high-temperature resistant and arc corrosion resistant conductive materials.

Preferably, the electrode is connected with the base through an active connection mechanism, so that the electrode can freely adjust the position in the lower area of the base.

More preferably, the electrode is connected with the base through an active connection mechanism, so that the electrode can be adjusted in position in the vertical direction and the horizontal direction.

Preferably, the active connection mechanism is vertically connected to the base.

More preferably, the active connection mechanism may also be connected to the base non-perpendicularly.

Preferably, the electrode is rotatably connected with the active connection mechanism, so that the electrode can rotate freely to adjust the angle.

More preferably, the electrode is rotatably connected with the active connection mechanism, so that the electrode can rotate to adjust an included angle with the vertical direction.

Preferably, the included angle $\theta$ in the extension line of the symmetry axis of each two arc surface electrodes 3 in the symmetrical position is 5°-160°.

Preferably, the gliding arc plasma reactor is used for methane conversion reaction, and the included angle 0 between the extension lines of the symmetry axes of each two arc surface electrodes 3 in the symmetrical position is 10°-90°, and more preferably 30°-60°.

The gliding arc plasma reactor provided by the present invention can enable the raw material gas to pass through the discharge area formed by the electrodes more intensively, thereby effectively increasing the gas flow passing through the discharge area and improving the conversion efficiency of reactants.

Preferably, the material forming the outer cylinder of the gliding arc plasma reactor is selected from at least one of an insulating material, a conductive material, and a conductive material provided with an insulating liner.

More preferably, the material forming the outer cylinder of the gliding arc plasma reactor is an insulating material or a conductive material provided with an insulating lining.

Further preferably, the insulating material forming the outer cylinder is selected from at least one of ordinary glass, quartz glass, and corundum.

In the present invention, on the premise of avoiding the contact between the electrode and the outer cylinder of the gliding arc plasma reactor, the material forming the outer cylinder can also be a conductive material.

In the present invention, the shape of the outer cylinder of the gliding arc plasma reactor is not particularly limited, and may be any shape that can provide a closed space for the reactor, such as a cylindrical shape, a rectangular shape, or any other shape that can achieve the aforementioned object of the present invention.

Preferably, a lower reaction zone capable of being filled with catalyst is provided downstream of the gliding arc plasma generator.

Preferably, the material forming the lower reaction zone is a metallic material.

More preferably, the lower reaction zone is tapered, which is more favorable for the distribution of the reaction gas.

Preferably, the proportional relationship among the length d2 of the discharge area, the length d4 of the spacing region and the height d5 of the lower reaction zone satisfies: 1: 0.1-0.8: 0.5-1.5; the length d4 of the spacing region represents the distance between the bottom of the discharge area and the top of the lower reaction zone.

The gliding arc plasma reactor provided by the present invention can be filled with a catalyst capable of catalyzing the hydrogenation conversion of alkyne to olefin, and the catalyst is preferably filled in the lower reaction zone of the reactor.

The gliding arc plasma reactor provided by the present invention can realize continuous and stable reaction under higher reactant conversion efficiency, and compared with a traditional process for preparing olefin from methane, no $CO_2$ is generated; the ignition and explosion risk is avoided; and the reactor is safer and more environmentally friendly.

As previously mentioned, the second aspect the present invention provides a method for converting methane by means of plasma, the method being carried out in a gliding arc plasma reactor as described in the first aspect, the method comprising:

introducing a reaction gas containing methane into the gliding arc plasma reactor under plasma discharge conditions to carry out a methane conversion reaction.

According to a particularly preferred embodiment, under plasma discharge conditions, a reaction gas containing methane is introduced into the gliding arc plasma reactor through the reactor inlet 1, so that the reaction gas passes through the discharge area formed by the arc surface electrodes 3 to carry out a methane conversion reaction, and the products obtained after the reaction are led out of the gliding arc plasma reactor through the product outlet 5.

The reaction conditions involved in converting methane into olefins in the gliding arc plasma reactor provided by the present invention are not particularly limited, and can be performed in various conditions involved in plasma conversion methods conventionally used in the art, and the conditions involved in converting methane into olefins are exemplified in the examples section of the present invention, and those skilled in the art should not be construed as limiting the present invention.

The gliding arc plasma reactor provided by the present invention has no particular limitation on the concentration of methane in the reaction gas at the reactor inlet, and for example, the concentration of methane in the gas may be 0.01-100 vol%, and may be, for example, 5 vol%, 10 vol%, 15 vol%, 20 vol%, 25 vol%, 30 vol%, 35 vol%, 40 vol%, 45 vol%, 50 vol%, 55 vol%, 60 vol%, 65 vol%, 70 vol%, 75 vol%, 80 vol%, 85 vol%, 90 vol%, 95 vol%.

According to another preferred embodiment, the method further comprises: the reaction gas passes through a discharge area formed by the arc surface electrode 3 and then passes through a lower reaction zone 4 to carry out methane conversion reaction.

Preferably, the lower reaction zone 4 is a reaction zone capable of being provided with a catalyst bed layer, and the flow rate of the reaction gas containing methane is such that the space velocity when passing through the lower reaction zone in the gliding arc plasma reactor is 1000-10000h$^{-1}$, and more preferably 5000-8000h$^{-1}$.

Preferably, the conditions of the methane conversion reaction comprise: the discharge voltage U1 is 1.0-5.0kV, and the discharge current is 100-3000mA; the proportional relationship between the flow rate V1 of the reaction gas passing through the minimum distance D2 between every two arc surface electrodes 3 at the symmetrical position and the discharge voltage U1 is as follows: V1: U1= 50-100: 1.

Preferably, the conditions of the methane conversion reaction include: the discharge voltage U1 is 2.0-5.0kV, and the discharge current is 1000-3000 mA.

Preferably, the proportional relationship between the flow rate V1 of the reaction gas passing through the minimum distance D2 between every two arc surface electrodes 3 at the symmetrical position and the discharge voltage U1 is as follows:V1: U1= 50-80: 1, more preferably V1: U1= 60-80: 1.

Preferably, the catalyst in the catalyst bed comprises a Ti oxide doped carrier and an active component loaded on the carrier, wherein the active component contains the first active component and the second active component, the first active component is selected from at least one of non-noble metals in the VIII group and metals in the IB group, the second active component is selected from at least one of noble metals in the VIII group, and the weight ratio of the first active component to the second active component calculated by metal elements is 0.1-200: 1.

More preferably, the molar ratio of L acid to B acid in the Ti oxide doped carrier is 0.1-50: 1.

Preferably, the Ti oxide doped carrier is selected from at least one of Ti oxide doped $Al_2O_3$, Ti oxide doped $SiO_2$, Ti oxide doped MgO, and Ti oxide doped molecular sieve.

Preferably, in the Ti oxide doped carrier, the doping amount of the Ti oxide is 0.1-10wt% based on the total weight of the carrier.

Preferably, the first active component is selected from at least one of Cu, Ag, Au, Ni and Fe. Preferably, the second active component is selected from at least one of Pt, Rh, Pd and Ir.

Preferably, the weight ratio of the first active component to the second active component calculated by the metal element is 0.1-10: 1.

Preferably, the weight ratio of the first active component to the total weight of the catalyst is 0.1-2: 100, and the weight of the first active component is calculated by metal elements.

According to a preferred embodiment, the reaction gas is a gas obtained by mixing methane with a carrier gas.

Preferably, the carrier gas is hydrogen.

Preferably, the feed rate of methane is 0.5-5.0L/min and the feed rate of hydrogen is 1.0-5.0L/min; more preferably, the feed rate of methane is 0.5-2.5L/min and the feed rate of hydrogen is 1.0-2.5L/min.

According to a preferred embodiment, the method further comprises: the product withdrawn from the product outlet 5 is subjected to the first separation to obtain an olefin product and the first gaseous feed comprising hydrogen and a carrier gas.

Preferably, the first gaseous material is recycled, with or without separation, back to the gliding arc plasma reactor to continuously carry out the methane conversion reaction.

Preferably, the conditions of the first separation include separation of the carrier gas and hydrogen from the olefin product using at least one selected from the group consisting of membrane separation, cryogenic separation, and pressure swing adsorption processes.

Preferably, the method further comprises: and carrying out the second separation on the system product to obtain a carbodiolefin and a carbon tetraolefins respectively.

Preferably, the second separation operation comprises separating the olefin products to obtain ethylene, ethane, carbon tetraolefins and carbon tetraalkane.

More preferably, the conditions of the second separation comprise separation by a rectification process.

Another preferred embodiment of the present application for converting methane to olefins using the gliding arc plasma reactor described above is provided below:

nitrogen gas is passed into the gliding arc plasma reactor from the reactor inlet to purge the discharge area of air and to draw gas from the product outlet. Then introducing a reaction gas containing methane into the gliding arc plasma reactor from the reactor inlet, switching on a high-voltage power supply after the gas flow of the reaction gas is stable, and forming a plasma discharge field between the electrodes by adjusting the voltage and the frequency. Reaction gas sequentially passes through a discharge area and a lower reaction zone formed by electrodes to respectively carry out ionization and hydrogenation reactions, and a product obtained after the reaction is led out of the gliding arc plasma reactor through a product outlet.

In the present invention, after the reaction gas passes through the discharge area formed by the electrodes, the reaction gas carries heat generated by discharge and reactants to enter the lower reaction zone, the heat can provide heat required by the catalyst bed layer in the lower reaction zone, the catalyst bed layer does not need to be additionally heated, and the low energy consumption can be reduced on the premise of not influencing the conversion efficiency.

The present invention will be described in detail below by way of examples.

In the following examples, the raw materials are all commercially available unless otherwise specified. The remaining process parameters and equipment parameters in each example are set to be the same unless otherwise specified.

In the following examples, the methane conversion, ethylene selectivity, ethane selectivity, acetylene selectivity, hydrocarbon selectivity above $C_3$, and carbon deposition are calculated according to the following formulas: methane conversion % = (amount of methane before reaction - amount of methane after reaction)/amount of methane before reaction $\times$100%; hydrocarbon ($C_nH_m$) product selectivity % = (amount of $C_nH_m$ after reaction)$\times$n/(amount of methane before reaction - amount of methane after reaction)$\times$100%, n = an integer 2 to 5; carbon deposition % = 1 - hydrocarbon ($C_nH_m$) product selectivity %, n = an integer from 2 to 5.

Preparation example 1

Dissolving palladium nitrate in deionized water to form a palladium nitrate solution (the palladium content is 18wt%), dissolving copper nitrate in deionized water to form a copper nitrate solution (the copper content is 30wt%), wherein the palladium nitrate solution and the copper nitrate solution are mixed according to the mixing ratio that the palladium loading accounts for 0.5wt% of the catalyst mass and the copper loading accounts for 1wt% of the catalyst mass, adopting a $TiO_2$-$Al_2O_3$ carrier, adopting an excess impregnation method, mixing the two solutions, impregnating for 12h, drying for 4h at 80°C by rotary evaporation, then further drying for 8h at 120 °C in an oven, then placing in a muffle furnace, and roasting for 5h at 450°C to obtain a catalyst 1, wherein the chemical composition of the catalyst is as follows:

Pd element content of 0.5wt%, Cu element content of 1wt%, and the balance of $TiO_2$-$Al_2O_3$; the particle size of the active component is 5.4nm, and the ratio of L acid to B acid is 15.6.

Examples 1 to 4 are all carried out using the process scheme shown in Figure 1.

In examples 1 to 4, the methane conversion reaction is performed using a gliding arc plasma reactor, and the electrode gliding arc generator used is a gliding arc plasma generator and the electrode used is a arc surface electrode.

Example 1

The specific structure and structural parameters of the reactor are as follows:

the reactor comprises a reactor inlet, an electrode gliding arc generator, a lower reaction zone and a product outlet, wherein the electrode gliding arc generator comprises a gas nozzle, an electrode, a base and an active connection mechanism;

the base of the electrode gliding arc generator is provided with two electrodes which are symmetrically distributed, so that a discharge area can be formed between the electrodes; the center of the base is provided with the gas nozzle which is communicated with a gas inlet pipeline of the reactor inlet, so that reaction gas can enter the gliding arc plasma reactor from the reactor inlet through the gas nozzle;

the electrode is connected with the base through an active connection mechanism, so that the position of the electrode can be adjusted in the vertical direction and the horizontal direction; the active connection mechanism is vertically connected with the base; the electrode is rotatably connected with the active connection mechanism, so that the electrode can rotate to adjust an included angle with the vertical direction;

the electrode is a semi-closed arc surface electrode, the central angle $\alpha$ corresponding to the arc surface is 90° , and the material for forming the electrode is zinc-copper alloy (the main components of the zinc-copper alloy are 60wt% of copper, 37wt% of zinc, 1wt% of titanium, 1wt% of graphene and 1wt% of other materials);

the included angle $\theta$ in the extension line of the symmetry axis of two electrodes in the symmetrical position is 77° ;

the outer cylinder of the gliding arc plasma reactor is made of quartz glass;

the thickness of the catalyst bed layer enables the space velocity when the catalyst passes through the feed gas to be 2000 $h^{-1}$;

the volume of the gliding arc plasma reactor in this example is 3L.

The operating conditions of the gliding arc plasma reactor in this example are as follows:

the discharge power is adjusted to 300W, the voltage is 3.0kV, the discharge frequency is 22.3kHz, the gas inlet flow is 1L/min of methane and 3L/min of hydrogen; the height of a catalyst bed layer filled in a lower reaction zone of the reactor is 15mm, and the dosage of the catalyst is 60 g; the catalyst is catalyst 1 prepared in preparation example 1;

introducing nitrogen into the reactor through the reactor inlet for 30min, wherein the gas inflow is 3L/min, replacing oxygen in the reactor, introducing mixed gas (the gas inflow is 2L/min of nitrogen and 1L/min of hydrogen), starting a power supply, adjusting the voltage and the frequency, adjusting the voltage to 2.0kV, adjusting the frequency to 20 kHz, starting discharging, reducing the catalyst in the lower reaction zone for about 3h, and finishing the reduction, wherein the color of the catalyst basically turns black; turning off a power supply, introducing mixed gas (the gas inflow is 1L/min methane and 3L/min hydrogen), turning on the power supply, adjusting the voltage and the frequency, adjusting the voltage to 2.0kV, adjusting the frequency to 22.3kHz, starting discharging, adjusting the voltage to 3.0kV, and the power is 300W at this time, and reacting for 8 h;

the tail gas is analyzed, and the result is as follows: the conversion rate of methane is 47.9%, the selectivity of ethylene is 88.7%, the selectivity of ethane is 5.9%, the selectivity of hydrocarbons above $C_3$ is 5.4%, and no obvious carbon deposition exists.

Example 2

This example uses a gliding arc plasma reactor similar to that of example 1 for the methane conversion reaction, except that in this example:

the electrode is a semi-closed arc surface electrode, the central angle $\alpha$ corresponding to the arc surface is 120° , and the material for forming the electrode is 316L stainless steel;

the included angle $\theta$ in the extension line of the symmetry axis of two electrodes in the symmetrical position is 46° ;

the outer cylinder of the gliding arc plasma reactor is made of 304 stainless steel with a quartz lining;

the thickness of the catalyst bed layer enables the space velocity when the catalyst passes through the feed gas to be 6000 $h^{-1}$;

the volume of the gliding arc plasma reactor in this example is 4L.

In this example, the discharge power is adjusted to 250W, the voltage is 1.8kV, the discharge frequency is 25.5kHz, the gas inlet flow is 0.5L/min of methane and 2L/min of hydrogen;

introducing nitrogen into the reactor through the reactor inlet for 30min, wherein the gas inflow is 3L/min, replacing oxygen in the reactor, introducing mixed gas (the gas inflow is 2.5L/min of nitrogen and 1.5L/min of hydrogen), starting a power supply, adjusting voltage and frequency, adjusting voltage to 3.0kV and frequency to 16.3 kHz, starting discharging, reducing the catalyst in the lower reaction zone for about 2.5 h, and finishing the reduction, wherein the color of the catalyst basically turns black; turning off a power supply, introducing mixed gas (the gas inflow is 0.5L/min of methane and 2L/min of hydrogen), turning on the power supply, adjusting the voltage and the frequency, adjusting the voltage to 1.5 kV, adjusting the frequency to 25.5kHz, starting discharging, adjusting the voltage to the specified 1.8kV, and the power is 250W at this time, and reacting for 8 hours.

The rest of the process is the same as in example 1.

The tail gas is analyzed, and the result is as follows: the conversion rate of methane is 48.7%, the selectivity of ethylene is 89.2%, the selectivity of ethane is 4.7%, the selectivity of hydrocarbons above $C_3$ is 6.1%, and no obvious carbon deposition exists.

Example 3

This example uses a gliding arc plasma reactor similar to that of example 1 for the methane conversion reaction, except that in this example:

the electrode is a fully-closed arc surface electrode, and the material forming the electrode is graphite;
the included angle $\theta$ in the extension line of the symmetry axis of two electrodes at the symmetrical position is 15° ;
the outer cylinder of the gliding arc plasma reactor is made of toughened glass;
the thickness of the catalyst bed layer enables the space velocity when the catalyst passes through the feed gas to be 8000 $h^{-1}$;
the volume of the gliding arc plasma reactor in this example is 3.3L.

In this example, the discharge power is adjusted to 280W, the voltage is 3.5kV, the discharge frequency is 17.5kHz, the gas inlet flow is 1.75L/min of methane and 2.3L/min of hydrogen;
introducing nitrogen into the reactor through the reactor inlet for 30min, wherein the gas inflow is 3L/min, replacing oxygen in the reactor, introducing mixed gas (the gas inflow is 3L/min of nitrogen and 2.5L/min of hydrogen), starting a power supply, adjusting the voltage and the frequency, adjusting the voltage to 3.0kV and the frequency to 21.2kHz, starting discharging, reducing the catalyst in the lower reaction zone for about 3.5h, and finishing the reduction, wherein the color of the catalyst basically turns black; and turning off a power supply, introducing mixed gas (the gas inflow is 2.5L/min of methane and 2.5L/min of hydrogen), turning on the power supply, adjusting the voltage and the frequency, adjusting the voltage to 2.5 kV, adjusting the frequency to 17.5kHz, starting discharging, adjusting the voltage to the specified 3.5 kV, and the power is 280W at this time, and reacting for 8 hours.

The rest of the process is the same as in example 1.

The tail gas is analyzed, and the result is as follows: the conversion rate of methane is 47.5%, the selectivity of ethylene is 91.3%, the selectivity of ethane is 5.6%, the selectivity of hydrocarbons above $C_3$ is 3.1%, and no obvious carbon deposition exists.

Example 4

This example uses a gliding arc plasma reactor similar to that of example 1 for the methane conversion reaction, except that in this example:

the electrode is a semi-closed arc surface electrode, the central angle $\alpha$ corresponding to the arc surface is 72° , and the material for forming the electrode is nickel-chromium alloy (the main components of the nickel-chromium alloy are 10wt% of iron, 17 wt% of chromium, 1wt% of aluminum, 2wt% of titanium, 1wt% of niobium, 0.5wt% of molybdenum, 0.1wt% of tungsten, 0.2wt% of silicon, 0.05wt% of carbon, 0.05wt% of zirconium and the balance of nickel);
the included angle $\theta$ in the extension line of the symmetry axis of two electrodes in the symmetrical position is 40° ;
the outer cylinder of the gliding arc plasma reactor is made of ceramic;
the thickness of the catalyst bed layer enables the space velocity when the catalyst passes through the feed gas to be 3500 $h^{-1}$;
the volume of the gliding arc plasma reactor in this example is 5L.

In this example, the discharge power is adjusted to 350W, the voltage is 4.2kV, the discharge frequency is 25.5kHz, the gas inlet flow is 2.9L/min of methane and 3.8L/min of hydrogen;
introducing nitrogen into the reactor through the inlet of the reactor for 30min, wherein the gas inflow is 2.5L/min, replacing oxygen in the reactor, introducing mixed gas (the gas inflow is 2.5L/min of nitrogen and 1.5L/min of hydrogen), starting a power supply, adjusting voltage and frequency, adjusting voltage to 1.6kV and frequency to 20.4 kHz, starting discharging, reducing the catalyst in the lower reaction zone for about 4h, and finishing the reduction, wherein the color of the catalyst basically turns black; and turning off a power supply, introducing mixed gas (the gas inflow is 2.9L/min of methane and 3.8L/min of hydrogen), turning on the power supply, adjusting the voltage and the frequency, adjusting the voltage to 2.5 kV, adjusting the frequency to 25.5kHz, starting discharging, adjusting the voltage to specified 4.2 kV, and the power is 350W at this time, and reacting for 8 hours.

The rest of the process is the same as in example 1.

The tail gas is analyzed, and the result is as follows: the conversion rate of methane is 48.7%, the selectivity of ethylene is 90.5%, the selectivity of ethane is 6.7%, the selectivity of hydrocarbons above $C_3$ is 2.8%, and no obvious carbon deposition exists.

Examples 5 to 8 are all carried out using the process scheme shown in Figure 2.

In examples 5 to 8, the methane conversion reaction is carried out using a gliding arc plasma reactor, using a tubular electrode gliding arc generator as the electrode gliding arc generator, using a tubular electrode as the electrode, and the average thickness of the electrode material (excluding any coating layer that may be present) is 2 mm.

Example 5

The specific structure and structural parameters of the reactor are as follows:

the reactor comprises a reactor inlet, an electrode gliding arc generator, a lower reaction zone and a product outlet, wherein the electrode gliding arc generator comprises a gas nozzle, an electrode, a base and an active connection mechanism;

the base of the electrode gliding arc generator is provided with two electrodes which are symmetrically distributed, so that a discharge area can be formed between the electrodes; the gas nozzle is arranged in the relative direction of the electrode and is communicated with a gas inlet pipeline of the reactor inlet, so that reaction gas can enter the gliding arc plasma reactor from the reactor inlet through the gas nozzle;

the electrode is connected with the base through an active connection mechanism, so that the position of the electrode can be adjusted in the vertical direction and the horizontal direction; the active connection mechanism is vertically connected with the base; the electrode is rotatably connected with the active connection mechanism, so that the electrode can rotate to adjust an included angle with the vertical direction;

arranging the gas nozzles in a mutually staggered manner in the relative direction of the electrodes;

the material for forming the electrode is nickel-copper alloy (the main composition of the nickel-copper alloy is 0.15wt% of silicon, 7.0wt% of manganese, 0.15wt% of phosphorus, 22.0wt% of nickel, 3.0wt% of cobalt, 3.0wt% of zinc, 0.20wt% of lead, 3.0wt% of iron, 0.15wt% of rare earth elements, 0.006wt% of arsenic and the balance of copper);

the included angle $\theta$ in the extension line of the symmetry axis of two electrodes at the symmetrical position is 35° ;

the outer cylinder of the gliding arc plasma reactor is made of quartz glass;

the thickness of the catalyst bed layer enables the space velocity when the catalyst passes through the feed gas to be 2000h$^{-1}$;

the volume of the gliding arc plasma reactor in this example is 3L.

The operating conditions of the gliding arc plasma reactor in this example are as follows:

the discharge power is adjusted to 230W, the voltage is 2.7kV, the discharge frequency is 25.7kHz, the gas inlet flow is 1.3L/min of methane and 2.1L/min of hydrogen; the height of a catalyst bed layer filled in a lower reaction zone of the reactor is 15mm, and the dosage of the catalyst is 60g; the catalyst is catalyst 1 prepared in preparation example 1;

introducing nitrogen into the reactor through the inlet of the reactor for 30min, wherein the gas inflow is 3L/min, replacing oxygen in the reactor, introducing mixed gas (the gas inflow is 1.8L/min of nitrogen and 1.5L/min of hydrogen), starting a power supply, adjusting voltage and frequency, adjusting voltage to 2.0kV and frequency to 25.7kHz, starting discharging, reducing the catalyst in the lower reaction zone for about 3.5h, and finishing the reduction, wherein the color of the catalyst basically turns black; turning off a power supply, introducing mixed gas (the gas inflow is 1.3L/min of methane and 2.1L/min of hydrogen), turning on the power supply, adjusting the voltage and the frequency, adjusting the voltage to 2.0kV, adjusting the frequency to 25.7kHz, starting discharging, adjusting the voltage to the specified 2.7kV, and the power is 230W at this time, and reacting for 8 hours.

the tail gas is analyzed, and the result is as follows: the conversion rate of methane is 50.4%, the selectivity of ethylene is 89.3%, the selectivity of ethane is 6.5%, the selectivity of hydrocarbons above $C_3$ is 4.2%, and no obvious carbon deposition exists.

Example 6

This example uses a gliding arc plasma reactor similar to that of example 5, except that in this example:

arranging the gas nozzles in a mutually aligned manner in relative directions of the electrodes;
the material forming the electrode is 316L stainless steel;
the included angle θ in the extension line of the symmetry axis of two electrodes at the symmetrical position is 63° ;
the outer cylinder of the gliding arc plasma reactor is made of 304 stainless steel with a quartz lining;
the thickness of the catalyst bed layer enables the space velocity when the catalyst passes through the feed gas to be 6000 $h^{-1}$;
the volume of the gliding arc plasma reactor in this example is 4L.

In this example, the discharge power is adjusted to 257W, the voltage is 1.9kV, the discharge frequency is 14.9kHz, the gas inlet flow is 2.1L/min of methane and 2.8L/min of hydrogen;
introducing nitrogen into the reactor through the reactor inlet for 30min, wherein the gas inflow is 3L/min, replacing oxygen in the reactor, introducing mixed gas (the gas inflow is 2.2L/min of nitrogen and 2.8L/min of hydrogen), starting a power supply, adjusting voltage and frequency, adjusting voltage to 2.0kV and frequency to 14.9kHz, starting discharging, reducing the catalyst in the lower reaction zone for about 4.0 h, and finishing the reduction, wherein the color of the catalyst basically turns black; turning off a power supply, introducing mixed gas (the gas inflow is 2.1L/min of methane and 2.8L/min of hydrogen), turning on the power supply, adjusting the voltage and the frequency, adjusting the voltage to 1.5 kV, adjusting the frequency to 14.9kHz, starting discharging, adjusting the voltage to the specified 1.9kV, and the power is 257W at this time, and reacting for 8 hours.
The rest of the process is the same as in example 5.
The tail gas is analyzed, and the result is as follows: the conversion rate of methane is 51.2%, the selectivity of ethylene is 90.1%, the selectivity of ethane is 5.4%, the selectivity of hydrocarbons above $C_3$ is 3.3%, and no obvious carbon deposition exists.

Example 7

This example uses a gliding arc plasma reactor similar to that of example 5, except that in this example:

arranging the gas nozzles in a mutually aligned manner in relative directions of the electrodes;
the material forming the electrode is tungsten-cerium alloy ($Ce_xY_yCs_zW_mO_3$, wherein x: y: z: m: =0.1: 0.1: 0.5: 1);
the included angle θ in the extension line of the symmetry axis of two electrodes at the symmetrical position is 85° ;
the outer cylinder of the gliding arc plasma reactor is made of toughened glass;
the thickness of the catalyst bed layer enables the space velocity when the catalyst passes through the feed gas to be 8000 $h^{-1}$;
the volume of the gliding arc plasma reactor in this example is 3.3L.

In this example, the discharge power is adjusted to 149W, the voltage is 2.5kV, the discharge frequency is 26.5kHz, the gas inlet flow is 1.1L/min of methane and 1.6L/min of hydrogen;
introducing nitrogen into the reactor through the reactor inlet for 30min, wherein the gas inflow is 3L/min, replacing oxygen in the reactor, introducing mixed gas (the gas inflow is 2.5L/min of nitrogen and 1.6L/min of hydrogen), starting a power supply, adjusting voltage and frequency, adjusting voltage to 2.0kV and frequency to 26.5kHz, starting discharging, reducing the catalyst in the lower reaction zone for about 4.5 h, and finishing the reduction, wherein the color of the catalyst basically turns black; and turning off a power supply, introducing mixed gas (the gas inflow is 1.1L/min of methane and 1.6L/min of hydrogen), turning on the power supply, adjusting the voltage and the frequency, adjusting the voltage to 2kV, adjusting the frequency to 26.5kHz, starting discharging, adjusting the voltage to the specified 2.5kV, and the power is 149W at this time, and reacting for 8 hours.
The rest of the process is the same as in example 5.
The tail gas is analyzed, and the result is as follows: the conversion rate of methane is 52.1%, the selectivity of ethylene is 88.4%, the selectivity of ethane is 6.7%, the selectivity of hydrocarbons above $C_3$ is 4.9%, and no obvious carbon deposition exists.

Example 8

This example uses a gliding arc plasma reactor similar to that of example 5, except that in this example:
the reverse side of the outer surfaces of the two electrodes (half of the electrode circumference) are provided with a coating, while the opposite faces (the other half of the electrode circumference) are not provided with a coating.

The coating is carried out as described in the preferred embodiment above, with the $Al_2O_3$-ZnO-$HfO_2$ films applied sequentially from the bottom, with 100 single atomic layer deposited metal oxide thin films per coating.

The rest of the process is the same as in example 5.

The tail gas is analyzed, and the result is as follows: the conversion rate of methane is 52.3%, the selectivity of ethylene is 93.3%, the selectivity of ethane is 2.3%, the selectivity of hydrocarbons above $C_3$ is 4.4%, and no obvious carbon deposition exists.

From the results, when the gliding arc plasma reactor provided by the present invention is used for converting methane to generate olefin, the conversion rate of methane can be obviously improved, the selectivity of ethylene in the product can be improved, and carbon deposition can be obviously reduced compared with the prior art. The reactor provided by the present invention can realize continuous and stable reaction under higher reactant conversion efficiency, and compared with a traditional process for preparing olefin from methane, no $CO_2$ is generated; the ignition and explosion risk is avoided; and the reactor is safer and more environmentally friendly.

The preferred embodiments of the present invention have been described above in detail, but the present invention is not limited thereto. Within the scope of the technical idea of the present invention, many simple modifications can be made to the technical solution of the invention, including various technical features being combined in any other suitable way, and these simple modifications and combinations should also be regarded as the disclosure of the invention, and all fall within the scope of the invention.

**Claims**

1. A gliding arc plasma reactor, wherein the reactor comprising a reactor chamber and a gliding arc plasma generator disposed in the reactor chamber;
   the gliding arc plasma generator comprises at least two arc surface electrodes (3) which are symmetrically distributed, the discharge surfaces of each arc surface electrode are all arc surface structures, the central angle corresponding to each arc surface electrode is $\alpha$, wherein $360° \geq \alpha >5°$, preferred $360° \geq \alpha >10°$; the arrangement positions of the arc surface electrodes enable a discharge area to be formed between the arc surface electrodes (3).

2. The gliding arc plasma reactor according to claim 1, wherein the arc surface electrode (3) is a semi-closed arc surface structure, and $270° > \alpha >30°$;
   preferably, $\alpha$ is selected from at least one of $180°$, $120°$, $90°$, $72°$, $60°$.

3. The gliding arc plasma reactor according to claim 1, wherein the arc surface electrode (3) is a fully-closed arc surface structure.

4. The gliding arc plasma reactor according to claim 3, wherein the arc surface electrode (3) is a rod-shaped electrode or a hollow tubular electrode.

5. The gliding arc plasma reactor according to any one of claims 1 to 4, wherein the proportional relationship between the length d2 of the discharge area and the height d3 of the reactor chamber satisfies:

$$1: 1.2\text{-}1.8.$$

6. The gliding arc plasma reactor according to any one of claims 1 to 4, wherein the gliding arc plasma generator comprises two arc surface electrodes (3) or six arc surface electrodes (3) which are symmetrically distributed; and/or, a gas nozzle (2) is arranged at the center of the upper part of the gliding arc plasma generator, and the gas nozzle (2) is communicated with a gas inlet pipeline of a reactor inlet (1) of the reactor chamber.

7. The gliding arc plasma reactor according to any one of claims 1 to 4, wherein at least a plurality of gas nozzles (2) are arranged in the relative direction of each two cambered electrodes (3) in symmetrical positions, and each gas nozzle (2) is communicated with a gas inlet pipeline of the reactor inlet (1);
   preferably, on each arc surface electrode (3), the proportional relation between the distance between two adjacent gas nozzles (2) and the inner diameter r2 of gas nozzle satisfies the following conditions: 1: 0.5-1.5.

8. The gliding arc plasma reactor according to claim 6 or 7, wherein the proportional relationship between the inner diameter r2 of the gas nozzle (2) and the minimum width r3 of the discharge area satisfies: 1: 2-8;

preferably, the material forming the gas nozzle (2) is a conductive material;
preferably, the electrical conductivity of the conductive material forming the gas nozzle (2) is > 1MS/m, preferably >10MS/m; the thermal conductivity is >10W/(m.°C), preferably >50W/(m.°C).

9. The gliding arc plasma reactor according to any one of claims 1 to 4, wherein each arc surface electrode (3) is arranged obliquely, and the included angle θ in the extension line of the symmetry axis of each two arc surface electrodes 3 in the symmetrical position is 5° -160° ; preferably 10° -90° , more preferably 30° -60° .

10. The gliding arc plasma reactor according to any one of claims 1 to 4, wherein, in the reactor chamber, a lower reaction zone capable of being filled with a catalyst is provided downstream of the gliding arc plasma generator;

preferably, the lower reaction zone is tapered;
preferably, the proportional relationship among the length d2 of the discharge area, the length d4 of the spacing region and the height d5 of the lower reaction zone satisfies: 1: 0.1-0.8: 0.5-1.5; the length d4 of the spacing region represents the distance between the bottom of the discharge area and the top of the lower reaction zone.

11. A method for converting methane by means of plasma, wherein, the method being carried out in a gliding arc plasma reactor according to any one of claims 1 to 10, the method comprising:
introducing a reaction gas containing methane into the gliding arc plasma reactor under plasma discharge conditions to carry out a methane conversion reaction.

12. Method according to claim 11, wherein the flow rate of the reaction gas containing methane is such that the space velocity when passing through the lower reaction zone in the gliding arc plasma reactor is $1000$-$10000h^{-1}$, and preferably $5000$-$8000h^{-1}$.

13. Method according to claim 11 or 12, wherein the conditions of the methane conversion reaction comprise: the discharge voltage U1 is 1.0-5.0kV, and the discharge current is 100-3000mA; and/or,
the proportional relation between the flow rate V1 of the reaction gas containing methane passing through the top of a discharge area and the discharge voltage U1 is as follows: V1: U1= 50-100: 1, the unit of V1 is L/min, and the unit of U1 is kV.

14. Method according to claim 13, wherein the conditions of the methane conversion reaction comprise: the discharge voltage U1 is 2.0-5.0kV, and the discharge current is 1000-3000mA; and/or,
V1: U1= 50-80: 1, preferably, V1: U1= 60-80: 1.

15. Method according to any one of claims 11 to 14, wherein the catalyst packed in the lower reaction zone in the gliding arc plasma reactor comprises a Ti oxide doped carrier and an active component supported on the carrier;
the active component contains the first active component and the second active component, the first active component is selected from at least one of non-noble metals in the VIII group and metals in the IB group, the second active component is selected from at least one of noble metals in the VIII group.

16. Method according to claim 15, wherein the content weight ratio of the first active component element to the second active component element calculated by metal elements is 0.1-200: 1, preferably 0.1-10: 1.

17. Method according to claim 15, wherein the molar ratio of L acid to B acid in the Ti oxide doped carrier is 0.1-50: 1.

18. Method according to claim 15, wherein the Ti oxide doped carrier is selected from at least one of Ti oxide doped $Al_2O_3$, Ti oxide doped $SiO_2$, Ti oxide doped MgO, and Ti oxide doped molecular sieve; and/or,
in the Ti oxide doped carrier, the doping amount of the Ti oxide is 0.1-10wt% based on the total weight of the carrier.

19. Method according to any one of claims 15 to 18, wherein the first active component element is selected from at least one of Cu, Ag, Au, Ni, and Fe; and/or,
the second active component element is selected from at least one of Pt, Rh, Pd and Ir.

20. Method according to any one of claims 15 to 19, wherein the content of the first active component element in the catalyst is 0.1-2wt% calculated by metal element.

21. Method according to any one of claims 11 to 20, wherein the reaction gas containing methane is a mixture comprising methane and a carrier gas.

22. Method according to claim 21, wherein the carrier gas is hydrogen.

23. Method according to claim 22, wherein the methane and the carrier gas are separately fed via a pipeline, and the feed rate of methane is 0.5-5.0L/min and the feed rate of hydrogen is 1.0-5.0L/min;
preferably, the feed rate of the methane is 0.5-2.5L/min, and the feed rate of the hydrogen is 1.0-2.5L/min.

24. Method according to any one of claims 11 to 23, wherein the method further comprises: separating the product exiting the product outlet (5) of the gliding arc plasma reactor to obtain a carbodiolefin, a carbon tetraolefins and a gaseous feed that can be recycled to the reactor inlet (1) of the gliding arc plasma reactor.

Figure 1

Figure 2

## INTERNATIONAL SEARCH REPORT

| | International application No. |
|---|---|
| | **PCT/CN2022/140057** |

**A. CLASSIFICATION OF SUBJECT MATTER**

B01J 19/08(2006.01)i;C07C 2/80(2006.01)i;C07C 11/04(2006.01)i;H01J 37/32(2006.01)i;C23C 16/455(2006.01)i;C23C 16/40(2006.01)i;C07C 11/24(2006.01)i;C07C 2/84(2006.01)i;B01J 19/00(2006.01)i;H05H 1/34(2006.01)i;B01J 23/89(2006.01)i;B01J 23/42(2006.01)i;B01J 23/44(2006.01)i;B01J 23/46(2006.01)i;B01J 23/50(2006.01)i;B01J 23/52(2006.01)i;B01J 29/44(2006.01)i;B01J 23/745(2006.01)i

According to International Patent Classification (IPC) or to both national classification and IPC

**B. FIELDS SEARCHED**

Minimum documentation searched (classification system followed by classification symbols)

B01J C07C H01J C23C H05H

Documentation searched other than minimum documentation to the extent that such documents are included in the fields searched

Electronic data base consulted during the international search (name of data base and, where practicable, search terms used)

CNABS; CNTXT; ENTXTC; WPABSC; VEN; ENTXT; USTXT; WOTXT; EPTXT; CNKI; ISI Web of Knowledge; 万方, WANFANG: 石油化工, 石化安全, 催化, 载体, 触媒, 重整, 甲烷, 二氧化碳, 氢气, 滑动弧, 等离子体, 耦合, plasma, arc, discharge, gliding, CO2, H2, CH4, TI, AL2O3

**C. DOCUMENTS CONSIDERED TO BE RELEVANT**

| Category* | Citation of document, with indication, where appropriate, of the relevant passages | Relevant to claim No. |
|---|---|---|
| Y | US 2021121854 A1 (UNIVERSITY OF WYOMING) 29 April 2021 (2021-04-29) description, paragraphs 0008-0037 | 15-24 |
| A | WO 2014139664 A1 (AL-KO THERM GMBH) 18 September 2014 (2014-09-18) entire document | 1-24 |
| A | CN 107335386 A (DALIAN INSTITUTE OF CHEMICAL PHYSICS, CHINESE ACADEMY OF SCIENCES) 10 November 2017 (2017-11-10) entire document | 1-24 |
| X | ZAENAB, A. A. et al. "Plasma-catalytic dry reforming of methane in an atmospheric pressure AC gliding arc discharge" *Catalysis Today,* Vol. 256, No. 1, 08 May 2015 (2015-05-08), ISSN: 0920-5861, page 76, left column, paragraph 1-page 78, right column, paragraph 1, and figure 1 | 1-14, 21-24 |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **01 February 2023** | **24 February 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

EP 4 446 003 A1

**INTERNATIONAL SEARCH REPORT**

International application No.

**PCT/CN2022/140057**

**C. DOCUMENTS CONSIDERED TO BE RELEVANT**

| Category* | Citation of document, with indication, where appropriate, of the relevant passages | Relevant to claim No. |
|---|---|---|
| Y | ZAENAB, A. A. et al. "Plasma-catalytic dry reforming of methane in an atmospheric pressure AC gliding arc discharge" *Catalysis Today,* Vol. 256, No. 1, 08 May 2015 (2015-05-08), ISSN: 0920-5861, page 76, left column, paragraph 1-page 78, right column, paragraph 1, and figure 1 | 15-24 |

Form PCT/ISA/210 (second sheet) (July 2022)

20

**INTERNATIONAL SEARCH REPORT**
Information on patent family members

International application No.

**PCT/CN2022/140057**

| Patent document cited in search report | | | Publication date (day/month/year) | Patent family member(s) | | | Publication date (day/month/year) |
|---|---|---|---|---|---|---|---|
| US | 2021121854 | A1 | 29 April 2021 | EP | 3815781 | A1 | 05 May 2021 |
| | | | | JP | 2021070027 | A | 06 May 2021 |
| WO | 2014139664 | A1 | 18 September 2014 | DE | 102013004514 | B3 | 10 July 2014 |
| CN | 107335386 | A | 10 November 2017 | US | 2019143288 | A1 | 16 May 2019 |
| | | | | US | 10661239 | B2 | 26 May 2020 |
| | | | | EP | 3450419 | A1 | 06 March 2019 |
| | | | | EP | 3450419 | A4 | 04 December 2019 |
| | | | | WO | 2017185920 | A1 | 02 November 2017 |
| | | | | JP | 2019514673 | A | 06 June 2019 |
| | | | | JP | 6898352 | B2 | 07 July 2021 |
| | | | | CN | 107335386 | B | 22 January 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111567211X
- CN 202111565360
- CN 202111567220
- CN 202111567216
- CN 202111567218
- CN 202111565366X
- CN 202111565369
- CN 202111567199
- CN 202111567219
- CN 100999432 A
- CN 101734620 A
- CN 210367505 U
- CN 109294284 A
- CN 106478332 A
- CN 101921163 A
- CN 203582763 U
- CN 102068953 A
- CN 101550057 A
- CN 101734995 A
- CN 1613839 A
- CN 104056828 A
- CN 104056829 A
- CN 103333044 A
- CN 101844744 A

**Non-patent literature cited in the description**

- **THANYACHOTPAIBOON et al.** Conversion of methane to high hydrocarbons in AC nonequilibrium plasmas [J. *AIChE Journal,* 1998, vol. 44 (10), 2252-7
- **RUEANGJITT N et al.** Non-oxidative reforming of methane in a mini-gliding arc discharge reactor: Effects of feed methane concentration, feed flow rate, electrode gap distance, residence time, and catalyst distance [J. *Plasma Chem Plasma Process,* 2011, vol. 31 (4), 517-534